# EUROPÄISCHE PATENTANMELDUNG

(11) **EP 4 039 234 A1**
(43) Veröffentlichungstag der Anmeldung: **10.08.2022**
(21) Anmeldenummer: 21154902.7
(22) Anmeldetag: 03.02.2021
(51) Int. Cl.: A61F 7/02, A61F 7/08, H05B 6/02, A47J 39/02

(54) **MITTELS INDUKTION HEIZBARES WÄRMEKISSEN UND WÄRMEPAD HIERZU**

(71) Anmelder: Fallegger, Angela, 6055 Alpnach (CH); Aggeler, Patrick, 6055 Alpnach (CH)
(72) Erfinder: Fallegger, Angela, 6055 Alpnach (CH); Aggeler, Patrick, 6055 Alpnach (CH)
(74) Vertreter: Koelliker, Robert

(57) **Zusammenfassung**

Die vorliegende Erfindung betrifft ein mittels Induktion heizbares Wärmekissen (1) umfassend eine Aussenhülle (2) mit Oberseite (2a) und Unterseite (2b), ein Wärmemedium (3) und ein ferromagnetisches Element (4), wobei das Wärmemedium (3) durch die Aussenhülle (2) begrenzt ist und das ferromagnetische Element (4) im Wärmemedium (3) angeordnet ist, dadurch gekennzeichnet, dass das ferromagnetische Element (4)
- zumindest teilweise mit einem polymerhaltigen Material (5) umgeben ist,
- mindestens zwei übereinander angeordnete ferromagnetische Folien (4a) umfasst, die miteinander verbunden sind, und/oder
- in Form eines Textils vorliegt enthaltend sich berührende ferromagnetische Metallfasern (4b),
wodurch das ferromagnetische Element (4), gegebenenfalls zusammen mit dem Material (5), ein Wärmepad (6) bildet.

Beansprucht wird auch ein Wärmepad (6*) für das erfindungsgemässe Wärmekissen (1), ein Verfahren zum induktiven Erwärmen eines Wärmemediums (3) mit dem Wärmepad (6*), sowie die Verwendung des Wärmekissens (1).

## Beschreibung

Die vorliegende Erfindung bezieht sich auf ein mittels Induktion heizbares Wärmekissen umfassend ein Wärmepad, ein Wärmepad für Wärmekissen, ein Verfahren zum induktiven Erwärmen eines Wärmeelements mit dem Wärmepad, sowie die Verwendung des Wärmekissens.

Wärmen von Körperstellen gehören seit langem zu den Hausmitteln, um leichtere Verletzungen zu behandeln, Verspannungen zu lösen und das Wohlempfinden von Personen zu erhöhen. Dazu werden oft Wärmekissen eingesetzt, welche einen Wärmeträger beinhalten. Die Wärmekissen können beispielsweise Kerne von Steinobst enthalten, welche vor Gebrauch in einem Ofen erwärmt werden. Allerdings dürfen die Wärmekissen nicht zu stark erwärmt werden, um ein Brennen der Aussenhülle resp. ein Glühen der darin befindlichen Kerne zu verhindern.

Seit einiger Zeit sind auch Wärmebeutel im Einsatz, welche ein Medium enthalten, welches zumindest bei erhöhter Temperatur flüssig ist. Die Aussenhülle dieser Wärmebeutel ist typischerweise aus Kunststoff. Zur Erwärmung solcher Wärmebeutel eignen sich insbesondere heisses Wasser, da einerseits die Erwärmung relativ schnell geht und andererseits eine Überhitzung des Mediums deutlich über 100°C nicht möglich ist. Allerdings müssen die Wärmebeutel nach der Erwärmung aus dem heissen Wasser entfernt werden. Dabei muss darauf geachtet werden, dass die Wärmebeutel nicht mehr ins heisse Wasser rutschen, was zu gefährlichen Spritzern führen kann, welche die Haut verbrühen können. Zudem müssen die Wärmebeutel anschliessend mit einem Tuch getrocknet werden.

Alternativ können die Wärmebeutel in einem Ofen erwärmt werden, was jedoch eine gewisse Zeit braucht, da der Ofen nicht zu heiss eingestellt sein darf, um das Schmelzen des Kunststoffs zu verhindern.

Eine weitere Möglichkeit ist das Erwärmen der Wärmebeutel in einem Mikrowellenofen. Diese Art der Erwärmung ist jedoch besonders kritisch, da eine Temperatur der Wärmebeutel schwierig einstellbar ist und somit die Gefahr besteht, dass die Wärmebeutel überhitzen und dadurch platzen.

Mit der breiteren Anwendung von Induktionsherden wurden auch diverse Vorschläge gemacht, Wärmebeutel mittels Induktion zu erwärmen. So beschreibt die CN-A-1943528 eine Wärmeflasche mit elektromagnetischer Induktion und einem flexiblen Beutelkörper mit Flüssigkeitsinjektionsloch und Stopfen. Die Wärmeflasche enthält als Wärmemedium Wasser sowie eine magnetisch durchlässige Metallplatte. Dabei lässt sich mit dieser Wärmeflasche das Wasser elegant erwärmen. Allerdings besteht die Gefahr, dass anschliessend bei der Verwendung der Wärmeflasche die Metallplatte den flexiblen Beutelkörper verletzt, sodass das, gegebenenfalls heisse, Wasser austreten kann, was zu Verbrühungen führen kann. Zudem kann die Metallplatte bei unsachgemässer Handhabung zu Verletzungen, beispielsweise zu unerwünschten Druckstellen der Haut führen. Hinzu kommt, dass die Metallplatte starr ist und daher zwingend bei der Herstellung der Wärmeflasche in diese eingebaut werden muss. Ein Nachrüsten bestehender Wärmeflaschen mit der Metallplatte ist nicht möglich.

Es ist daher die Aufgabe der vorliegenden Erfindung, die Nachteile des Standes der Technik zu verbessern. Dabei ist es insbesondere wichtig, einen Wärmebeutel bereit zu stellen, welcher einfach erwärmt werden kann und auch bei unsachgemässer Handhabe zu keinen Verletzungen führt. Idealerweise weist er während dem Erwärmen eine gewisse Selbstregulation auf, um eine Überhitzung des Wärmemediums zumindest zu verlangsamen. Auch soll der Wärmebeutel äusserst biegbar sein und sich der Umgebung, beispielsweise zu wärmenden Körperstellen, einfach anpassen. Zudem soll es möglich sein, den Wärmebeutel auch in Bereichen mit erhöhten Sicherheitsanforderungen, wie in Gefängnissen oder Flugzeugen, einzusetzen, d.h. der Wärmebeutel darf keine harten Kanten und Ecken aufweisen.

Diese Aufgabe konnte überraschenderweise gelöst werden mit einem mittels Induktion heizbaren Wärmekissen (1) umfassend eine Aussenhülle (2) mit Oberseite (2a) und Unterseite (2b), ein Wärmemedium (3) und ein ferromagnetisches Element (4), wobei das Wärmemedium (3) durch die Aussenhülle (2) begrenzt ist und das ferromagnetische Element (4) im Wärmemedium (3) angeordnet ist, dadurch gekennzeichnet, dass das ferromagnetische Element (4)
- zumindest teilweise mit einem polymerhaltigen Material (5) umgeben ist,
- mindestens zwei übereinander angeordnete ferromagnetische Folien (4a) umfasst, die miteinander verbunden sind, und/oder
- in Form eines Textils vorliegt enthaltend sich berührende ferromagnetische Metallfasern (4b),
wodurch das ferromagnetische Element (4), gegebenenfalls zusammen mit dem Material (5), ein Wärmepad (6) bildet.

Beansprucht wird auch ein Wärmepad (6*) für die erfindungsgemässen Wärmekissen (1), wobei das Wärmepad (6*)
- ein ferromagnetisches Element (4) in Form von mindestens zwei, übereinander angeordneten ferromagnetischen Folien (4a) umfasst, wobei der Randbereich der Folien (4a) mit mindestens je einer Schicht des Materials (5) zumindest teilweise umfasst ist und das Material (5) und die Folien (4a) punktuell oder vollständig miteinander verbunden sind, oder
- ein Textil umfasst mit sich berührenden ferromagnetischen Metallfasern (4b).

Zudem wird auch ein Verfahren zum induktiven Erwärmen eines Wärmemediums (3) mit dem erfindungsgemässen Wärmepad (6*) beansprucht, wobei
- das Wärmepad (6*) in eine Aussenhülle (2) gebracht wird, wobei vorher, nachher und/oder gleichzeitig Wärmemedium (3) in die Aussenhüllte (2) gefüllt wird, und
- die Aussenhülle (2) mit Wärmepad (6*) und Wärmemedium (3) auf einer Induktionsheizplatte, insbesondere mittels Anlegen eines Wechselstroms an der Induktionsheizplatte, erwärmt wird, wobei
- die Aussenhülle (2), gegebenenfalls verschlossen wird, wodurch so das erfindungsgemässe Wärmekissen (1) erhalten wird.

Beansprucht wird zudem auch die Verwendung des erfindungsgemässen Wärmekissens (1) zum Auftauen, Wärmen und/oder Warmhalten von Substraten wie Lebensmittel, zum Wärmen und/oder Warmhalten von Körperstellen von lebenden Körpern, und/oder zur Wärmebehandlung in Spitälern, medizinischen Praxen und/oder im privaten Bereich, wobei das Wärmekissen (1) gegebenenfalls auch mit einer Schutzhülle umgeben sein kann.

Die erfindungsgemässen Wärmekissen (1), Wärmepad (6*), Verfahren und Verwendung weisen überraschenderweise viele Vorteile auf. So können das Wärmekissen (1) und das Wärmepad (6*) besonders einfach und sicher auf einem Induktionsherd ohne heisses Wasser erwärmt werden, was auch bei unsachgemässer Handhabe zu keinen Verletzungen führt, da der Induktionsherd selbst nicht heiss ist und das Wärmekissen (1) und das Wärmepad (6*) keine scharfen Kanten und Ecken aufweisen. Dies ist u.a. besonders bei Personen mit einer körperlichen Beeinträchtigung von grossem Vorteil, beispielsweise bei Personen ohne Wärmeempfinden oder mit sehr empfindlicher Haut. Zudem ist das Wärmekissen (1) und das Wärmepad (6*) äusserst biegbar und passen sich der Umgebung, beispielsweise den zu wärmenden Körperstellen, einfach an. Daher kann auch bei empfindlicher Haut kein Dekubitus entstehen - auch wenn eine Person irrtümlich über längere Zeit auf dem Wärmepad (6*) liegt.

Aufgrund der mangelnden scharfen und harten Kanten und Ecken kann das Wärmekissen (1) auch in Bereichen mit erhöhten Sicherheitsanforderungen, beispielsweise in Gefängnissen oder Flugzeugen, eingesetzt werden.

Auch ist es möglich, dass das Wärmekissen (1) mit einer Aussenhülle (2) in Form von Plastik zusammen mit dem Wärmemedium (3) und Wärmepad (6, 6*) auf den Induktionsherd gelegt werden kann und das Wärmemedium (3), beispielsweise Wasser, schnell erwärmt, und somit erhitzt werden kann. Dabei wird das Wärmekissen (1) nicht nass und kann sofort - und ohne Trocknen - verwendet werden.

Äusserst vorteilhaft ist zudem, dass das Wärmekissen (1) und das Wärmepad (6*) problemlos waschbar sind, da sie keine elektrischen Anschlüsse aufweisen. Zudem können sie erhöhten Temperaturen und Tensiden ausgesetzt werden. Somit können das Wärmekissen (1) und das Wärmepad (6*) auf einfache Art und Weise in die Waschmaschine oder in den Geschirrspüler gelegt und so gereinigt werden.

Das Wärmepad (6, 6*) besitzt eine relativ geringe Dichte und daher ein geringes Gewicht. Da das ferromagnetische Element (4, 4a, 4b) typischerweise flächig angeordnet ist, genügt schon ein relativ geringer Auftrieb, um das Wärmepad (6, 6*) innerhalb des Wärmemediums steigen zu lassen. Da sich das Wärmemedium (3) zuerst im Bereich der Oberflächen des Wärmepads (6, 6*) - und somit auch an der Unterseite des Wärmepads (6, 6*) und gegebenenfalls innerhalb des Wärmepads (6, 6*) - erwärmt, genügt schon eine geringe Erwärmung des Wärmemediums (3), um dem Wärmepad (6, 6*) einen Auftrieb zu geben. Dadurch steigt das Wärmepad (6, 6*) mit dem Erwärmen des Wärmemediums (3) nach oben. Übersteigt die Distanz des Wärmepads (6, 6*) zum Induktionskochfeld, d.h. zum Induktionsherd, die Reichweite der induktiven Erwärmung, wird die Erwärmung des Wärmepads (6, 6*), und somit des Wärmemediums (3), automatisch gestoppt. Dadurch wird ein zu schnelles Erhitzen des Wärmekissens (1) verhindert, was eine bessere Überwachung des Wärmevorgangs erlaubt. Somit wird das Erwärmen des Wärmemediums (3) im Wärmekissen mit dem Wärmepad (6, 6*) zumindest zum Teil selbst reguliert, wodurch eine allfällige Überhitzung des Wärmemediums (3), und somit des Wärmekissens (1), besser reguliert werden kann. Dieser Effekt wird verstärkt, wenn das Wärmepad (6, 6*) stark erwärmt wird, wodurch Wärmemedien (3) mit einem Siedepunkt von beispielsweise 80 bis 160°C, beispielsweise Wasser mit einem Siedepunkt von ca. 100°C bei Normaldruck, lokal verdampfen und sich stark ausdehnen können. Und wenn das Wärmepad (6, 6*) mindestens zwei übereinander angeordnete ferromagnetische Elemente (4) - insbesondere Folien (4a) - umfasst, wird das Wärmemedium (3) zwischen den Elementen (4) resp. Folien (4a) besonders schnell erhitzt, was zu schnellem lokalem Verdampfen des Wärmemediums (3) und zu starkem Auftrieb des Wärmepad (6, 6*) führt. Dies wiederum führt zu einer schnellen und hohen Empfindlichkeit in Bezug auf eine allfällige Überhitzung des Wärmemediums, was äusserst voreilhaft ist.

Das erfindungsgemässe Wärmepad (6*) ist überraschend äusserst biegbar. So kann es - wie auch das Wärmekissen (1) bei geeigneter Aussenhülle (2) - problemlos von Hand um ein Rohr mit einem Durchmesser von beispielsweise 10 cm reversibel gebogen werden. Dies ist nicht nur bei der Verwendung des Wärmekissens (1) von grossem Vorteil, sondern ermöglicht auch, dass Wärmekissen mit einer Öffnung, beispielsweise eine Bettflasche, mit dem Wärmepad (6*) auch nachträglich ausgestattet werden können. Dabei kann das Wärmepad (6*) einfach zusammengerollt und in die Öffnung des Wärmekissens eingefügt werden, wodurch das erfindungsgemässe Wärmekissen (1) erhalten wird. Zudem kann das Wärmepad (6*) aufgrund des niedrigen Gewichts und der hohen Biegbarkeit auch auf einfache Art und Weise im Reisegepäck mitgeführt werden, um bei Bedarf selbst ein Wärmekissen (1) herzustellen und zu erwärmen. Umfasst das Wärmepad (6*) ein Textil mit sich berührenden ferromagnetischen Metallfasern (4b), kann das Wärmepad (6*) als solches verwendet werden oder beispielsweise in Kleidungsstücke wie Handschuhe integriert werden. So können diese vor Gebrauch auf einfache Art und Weise auf einem Induktionsherd erwärmt werden.

Dadurch ist es mit dem erfindungsgemässen Verfahren möglich, ein Wärmemedium (3) mit dem erfindungsgemässen Wärmepad (6*) auf äusserst einfache Art und Weise zu erwärmen, sofern ein Induktionsherd mit Stromanschluss verfügbar ist. So genügt beispielsweise ein Plastiksack als Aussenhülle (2), in welchen als Wärmemedium (3) Wasser gefüllt wird, um das Wärmemedium (3) zu erwärmen. Dabei kann der Plastiksack beim Erwärmen offen bleiben oder vorher verschlossen werden. Somit ist eine Metallpfanne nicht mehr notwendig, um Wasser zu erwärmen. Dies kann beispielsweise besonders vorteilhaft sein beim Campieren.

### Das Wärmekissen (1)

Das mittels Induktion heizbare erfindungsgemässe Wärmekissen (1) umfasst eine Aussenhülle (2) mit Oberseite (2a) und Unterseite (2b), ein Wärmemedium (3) und ein ferromagnetisches Element (4). Das ferromagnetische Element (4) bildet, gegebenenfalls zusammen mit dem polymeren Material (5), ein Wärmepad (6). Dabei kann das Wärmekissen (1) das Wärmepad (6) oder das erfindungsgemässe Wärmepad (6*), gemeinsam Wärmepad (6, 6*) genannt, umfassen. Geeignete Wärmekissen (1) - und somit auch die eingesetzten Wärmemedien (3) lassen sich beispielsweise bis gegen 100°C erwärmen.

In einer Ausführungsform weist das Wärmekissen (1) eine beispielhafte Länge von 10 bis 40 cm, insbesondere von 15 bis 30 cm, eine Breite von 5 bis 30 cm, insbesondere von 10 bis 20 cm, sowie eine Höhe von 1 bis 10 cm, insbesondere 1 bis 5 cm auf.

Die Aussenhülle (2) kann neben der Oberseite (2a) und der Unterseite (2b) auch ein oder mehrere Seitenelemente, welche die Ober- und Unterseiten miteinander verbinden, aufweisen.

Die Oberseite (2a) und Unterseite (2b) der Aussenhülle (2) können identisch oder verschieden sein. Ist beispielsweise ein Flüssigkristall-Thermometer (7) und/oder eine bestimmte Markierung an einer Seite der Aussenhülle (2) angebracht, wird diese Seite typischerweise zur Oberseite (2a) und die gegenüberliegende Seite zur Unterseite (2b).

Die Aussenhülle kann eine verschliessbare Öffnung zum Befüllen des Wärmekissens (1) aufweisen. Durch diese Öffnung kann bei Bedarf auch das Wärmepad (6, 6*) in das Wärmekissen (1) eingefügt werden. Das Wärmemedium (3) ist durch die Aussenhülle (2) begrenzt und das ferromagnetische Element (4) ist im Wärmemedium (3) - und somit innerhalb der Aussenhülle (2) - angeordnet.

Das ferromagnetische Element (4) des Wärmekissens (1) kann in verschiedenen Ausführungsformen vorliegen und
- ist zumindest teilweise mit einem polymerhaltigen Material (5) umgeben, wobei das ferromagnetische Element (4) beispielsweise als Metallschicht, die gegebenenfalls profiliert sein kann, in Form von Folien (4a) oder in Form eines Textils enthaltend sich berührende ferromagnetische Metallfasern (4b), vorliegen,
- umfasst mindestens zwei, bevorzugt 2 bis 20, insbesondere 3 bis 10, übereinander angeordnete ferromagnetische Folien (4a), die miteinander verbunden sind, und/oder
- liegt in Form eines Textils vor enthaltend sich berührende, insbesondere miteinander verwobene und/oder verschweisste, ferromagnetische Metallfasern (4b),
wodurch das ferromagnetische Element (4), gegebenenfalls zusammen mit dem Material (5), ein Wärmepad (6) bildet.

Das ferromagnetische Element (4), die ferromagnetische Folie (4a) und/oder die ferromagnetischen Metallfasern (4b) des erfindungsgemässen Wärmekissens (1) basieren bevorzugt auf Eisen, Nickel, Cobalt, Chromdioxid und/oder einer ferromagnetischen Legierung, welche insbesondere auf AlNiCo, SmCo, Nd₂Fe₁₄B, Ni₈₀Fe₂₀, NiFeCo, Permalloy oder Mumetall, basiert. Somit können die Metalle und Legierungen auch weitere Elemente enthalten. Die ferromagnetische Folie (4a) kann gegebenenfalls als metallische Folie vorliegen, die beispielsweise faserverstärkt ist, und/oder in Form einer Kunststofffolie, welche mit einer ferromagnetischen Folie, beispielsweise einer selbstklebenden Eisenfolie, auch Ferrofolie genannt, beschichtet ist.

Indem das ferromagnetische Element (4) in Form einer ferromagnetischen Folie (4a) und/oder ferromagnetischen Metallfasern (4b) vorliegt, kann das ferromagnetische Element (4) ein - im Vergleich zur Oberfläche - geringes Gewicht aufweisen. Dadurch reagiert das Wärmepad (6, 6*) besonders empfindlich auf die induktive Erwärmung und steigt im flüssigen Wärmemedium (3) nach oben.

Das ferromagnetische Element (4) kann beispielsweise in rechteckiger, ovaler oder runder Form vorliegen und weist typischerweise eine Länge von mindestens 5 x 5 cm, insbesondere von mindestens 10 x 10 cm, resp. einen Durchmesser von mindestens 5 cm, insbesondere von mindestens 10 cm, auf.

Geeignete, gegebenenfalls faserverstärkte, ferromagnetische Folien (4a) sind dem Fachmann bekannt und im Handel erhältlich. Nicht-limitierende Beispiele von geeigneten Fasern zur Verstärkung der ferromagnetischen Folie (4a) umfassen Glasfasern, Kohlenstofffasern, Carbonfasern, Keramikfasern, Aramidfasern, Borfasern, Basaltfasern, Metallfasern, Stahlfasern, Naturfasern und/oder Nylonfasern.

Liegt das ferromagnetische Element (4) des Wärmepads (6, 6*) in Form eines Textils enthaltend sich berührende ferromagnetische Metallfasern (4b) vor, beträgt der Anteil Metallfasern (4b) im Textil typischerweise mindestens 10 Gew.-%, bevorzugt mindestens 30 Gew.-% und kann bis 80 Gew.-%, bevorzugt 70 Gew.-%, betragen, wobei jedoch auch 100 Gew.-% an Metallfasern (4b) möglich sind. Dabei ist das Textil bevorzugt ein Gewebe oder ein Vlies, wobei die Metallfasern (4b) sich berühren und dadurch miteinander verbunden sind. Dies wird durch eine geeignete Herstellung des Gewebes oder Vlies erzielt. Alternativ können die Metallfasern (4b) auch miteinander verschweisst oder verlötet sein. Dabei kann das Wärmepad (6, 6*) eine oder mehrere Schichten Textil umfassen. Bei mehreren Schichten werden diese vorteilhafterweise übereinander geschichtet miteinander verbunden, insbesondere miteinander vernäht.

Die weiteren Fasern des Textils umfassend Metallfasern (4b), d.h. Fasern welche zusammen mit den ferromagnetischen Metallfasern (4b) das Textil bilden, umfassen bevorzugt eine oder mehrere Naturfaser, insbesondere pflanzliche Faser, tierische Faser und/oder mineralische Faser; eine oder mehrere Chemiefaser, insbesondere Fasern aus natürlichen Polymeren wie Cellulosefasern, Wollfasern und/oder Baumwollfasern, synthetische Fasern, beispielsweise basierend auf Polyester, Polyamid, Aramid, Nylon, Polyurethan und/oder Elastan, und/oder Fasern wie Glasfasern, Kohlenstofffasern, Carbonfasern, Keramikfasern, Borfasern, Basaltfasern. Dabei werden Naturfaser und Fasern aus natürlichen Polymeren bevorzugt in Wärmepads (6, 6*) eingesetzt, welche zusammen mit einem rieselfähigen Wärmemedium (3) in einem Wärmekissen (1) verwendet werden. Bei Wärmemedien (3) die zumindest bei erhöhter Temperatur flüssig sind, werden bevorzugt synthetische Fasern und/oder anorganische Fasern wie Glasfaser, Kohlenstofffaser, Carbonfaser, Keramikfaser, Borfaser und/oder Basaltfaser eingesetzt.

In einer besonders bevorzugten Ausführungsform des Wärmekissens (1) und des Wärmepads (6*) sind die übereinander angeordneten ferromagnetischen Folien (4a) übereinander geschichtet und miteinander verbunden, bevorzugt punktuell und/oder im Randbereich, und insbesondere miteinander verschweisst, verklebt und/oder vernäht. Dabei können die Folien (4a) im Wärmepad (6) des Wärmekissens (1) auch ohne polymeres Material (5) vorliegen. Sind die Folien (4a) mittels Verklebung miteinander verbunden, kann diese auch mit dem polymerhaltigen Material (5) in Form von Kunststoffen, Silikonen, Siloxanen, Duromeren, Duroplasten, Kunstharze, Polyesterharze, Epoxidharze, Elastomeren und/oder Thermoplasten erfolgen, welche typischerweise in flüssiger Form um die Folien (4a) angebracht werden, wo sie aushärten können. Mit anderen Worten: Ein geeigneter Klebstoff kann nach dem Aushärten ein geeignetes polymeres Material (5) bilden.

Durch das Verbinden der übereinander geschichteten Folien (4a) werden diese stabiler und deshalb zerstörungsresistenter. Zudem kann dadurch bei geeigneter Anordnung Wärmemedium (3) zwischen die Folien (4a) gelangen, wodurch das Wärmemedium (3) von oben und unten - und somit besonders schnell - erwärmt wird. Dabei schützt das polymere Material (5), sofern vorhanden, den Randbereich der Folien (4a) vor mechanischen Einflüssen und reduziert zusätzlich die Dichte des Wärmepads (6, 6*), wodurch dieses weniger Auftrieb benötigt, um sich nach oben zu bewegen.

Die ferromagnetischen Folien (4a) weisen bevorzugt eine Dicke von je 0.01 bis 5 mm, bevorzugt von 0.05 bis 3 mm, insbesondere von 0.1 bis 1 mm, gemessen gemäss EN ISO 16809:2019, und/oder die ferromagnetischen Metallfasern (4b) einen Durchmesser von 0.2 µm bis 3 mm, insbesondere 0.5 µm bis 2 mm, auf. Dadurch kann das Gewicht des ferromagnetischen Elements - und somit des Wärmepads (6, 6*) niedrig gehalten werden.

Das optionale polymerhaltige Material (5), welches in einer Ausführungsform das ferromagnetische Material (4) des Wärmekissens (1) zumindest teilweise umgibt, ist bevorzugt auf Basis eines Polymers, Silikons; Siloxans, von Kunststoffen, Kork, Leder, und/oder Kautschuk, wobei das Polymer auch geschäumt und/oder faserverstärkt sein kann und bevorzugt ein Duromer, Duroplast, Kunstharz, Polyesterharz, Epoxidharz, Elastomer und Thermoplast umfasst, wobei Komposits enthaltend mindestens eines dieser Materialien sowie Derivate davon mitumfasst sind. Dabei kann das Polymer beispielsweise ein Polyamid (PA), Polyethylen (PE), sulfoniertes und/oder chlorsulfoniertes Polyethylen wie Hypalon^{®}, Ethylen-Propylen-Dien-Monomer Kautschuk (EPDM), Polypropylen (PP), Polyethylenterephthalat (PET), Polyvinylchlorid (PVC), Polyurethan (PU), sowie deren Misch-, Co- und Block(co)polymere, ein darauf basierendes Derivat und/oder Mischungen davon sein. Dabei werden für Anwendungen als Wärmekissen (1) zum Warmhalten von Körperstellen als Materialien (5) besonders bevorzugt, die bei Raumtemperatur biegbar sind, wie Silikone, Siloxane, Leder, Kautschuk und Elastomere besonders bevorzugt. Wird das Wärmekissen (1) bevorzugt zum Warmhalten von Lebensmittel eingesetzt, kann als Material (5) auch ein steifes Material, beispielsweise Kork oder Hart-PVC, eingesetzt werden.

Das polymerhaltige Material (5) ist bevorzugt flexibel und biegbar. Dies ist insbesondere wichtig, wenn das ferromagnetische Element, um welches das Material (5) angeordnet ist, übereinander angeordnete ferromagnetische Folien (4a) sind, wodurch das Wärmepad (6,6*) flexibel und biegbar bleibt.

In einer Ausführungsform kann das polymerhaltige Material (5) beispielsweise eine Breite von 0.1 bis 5 cm, insbesondere von 0.3 bis 2 cm, gemessen mit gemessen gemäss EN ISO 16809:2019, aufweisen und das ferromagnetische Element (4) umgeben.

Als Wärmemedium (3) für das Wärmekissen (1) kann eine Vielzahl an Substanzen eingesetzt werden. Besonders bevorzugte Wärmemedien (3) umfassen
- Wasser, wie Leitungswasser oder deionisiertes Wasser, oder eine wässrige Lösung, beispielsweis von anorganischen Verbindungen oder Mischungen, insbesondere Mischungen mit einem anorganischen Salz wie Kochsalz oder ein Salzhydrat;
- eine organische Verbindung mit Schmelzpunkt unterhalb 20°C, welche beispielsweise ein Molekulargewicht von 250 oder weniger aufweist, insbesondere ein Glykol, Polyethylenglykol, Polypropylenglykol und/oder ein Paraffin;
- eine organische Verbindungen mit Schmelzpunkt zwischen 20°C und 80°C, bevorzugt zwischen 20°C und 60°C, insbesondere zwischen 30°C und 50°C, wie z.B. Paraffine, Alkohol, Glykol, Polyol, Zucker, Keton, Ester, Ether, Carbonsäure, Fettsäure, Amid, eine Schwefel-, Phosphor- und/oder Stickstoff-Verbindung; und/oder Latentwärmespeichermaterialien, auch Phase Change Material (PCM) genannt, wobei diese vielseitig in der Literatur, beispielsweise im VDI Wärmeatlas, 10. Auflage, Springer Verlag, beschrieben sind und dem Fachmann bekannt sind;
- rieselfähige, feste Materialien wie Sand, Fruchtkerne wie Kerne von Steinobst wie Pfirsich, Kirschen, Nektarinen, Aprikosen, Zwetschgen, Pflaumen und/oder Mirabellen, Kernobst wie Äpfel und/oder Birnen, und/oder ein Textil, insbesondere ein Gewebe und/oder Vlies,
umfasst. Diese Wärmemedien (3) sind gut verfügbar und/oder weisen eine besonders grosse Wärmekapazität auf, wodurch das Wärmekissen (1) nach dem Erwärmen über einen langen Zeitraum warm bleibt.

Die Aussenhülle (2) des Wärmekissens (1) mit Oberseite (2a) und Unterseite (2b), sowie gegebenenfalls ein oder mehrere Seitenelemente, welche die Ober- und Unterseiten miteinander verbinden, basieren bevorzugt auf einem Polymer oder einem polymerbeschichteten Textil, Gewebe oder Vlies, wobei das Polymer und/oder die Polymerbeschichtung bevorzugt auf Basis von Polyethylen (PE), Polypropylen (PP), Polyethylenterephthalat (PET), Polyvinylchlorid (PVC), Polyvinylidenchlorid (PVDC), Ethylen-Propylen-Dien-Kautschuk (EPDM), Chloropren-Kautschuk (CR), Styrol-Butadien-Kautschuk (SBR), Polyurethan (PU), Polyamid (PA), deren Misch-, Co- und Block(co)polymere und/oder deren Derivate ist. Diese Materialien weisen eine hohe Flexibilität, Biegbarkeit, mechanische Beständigkeit auf und können schnell und einfach verschlossen, beispielsweise verschweisst, werden. Auch sind sie für die Verwendung des Wärmekissens (1) bis beispielsweise 100°C genügend temperaturbeständig.

Dabei wird vorteilhafterweise das Material der Aussenhülle (2) auf das Wärmemedium (3) und den Einsatzbereich abgestimmt, wobei kein Wärmemedium (3) durch die Aussenhülle (2) dringen soll. Während beispielsweise ein Textil als Aussenhülle (2) rieselfähige Wärmemedien (3) beinhalten kann, sind für Wärmemedien (3), welche bei Raumtemperatur flüssig sind oder bei erhöhter Temperatur flüssig werden, Polymere oder polymerbeschichtete Materialien vorteilhaft.

Soll das Wärmekissen (1) zum Warmhalten von Körperstellen eingesetzt werden können, ist es besonders bevorzugt, wenn die Aussenhülle (2) bei Raumtemperatur biegbar ist und daher beispielsweise auf Basis von Polyethylen (PE), Ethylen-Propylen-Dien-Kautschuk (EPDM), Chloropren-Kautschuk (CR) oder Polyurethan (PU). Wird das Wärmekissen (1) bevorzugt zum Warmhalten von Lebensmittel eingesetzt, kann als Material (5) auch ein steifes Material, beispielsweise Polypropylen (PP), Polyethylenterephthalat (PET), Hart-PVC oder Polyethylenterephthalat (PET), eingesetzt werden.

Werden als Aussenhülle (2) Polymere eingesetzt, weisen diese bevorzugt eine Erweichungstemperatur von mindestens 110°C auf. Dadurch sind sie stabil genug, um darin Wasser zu erwärmen, können jedoch auf einfache Art und Weise miteinander verschweisst werden, um die Oberseite (2a) und Unterseite (2b), sowie gegebenenfalls das oder die Seitenelemente, so miteinander zu verbinden, sodass keine Wärmeflüssigkeit (3) auch bei hoher mechanischer Beanspruchung austreten kann. Geeignete Schweissmethoden umfassen Thermoschweissen und Ultraschallschweissen.

Die Schichtdicke der Aussenhülle (2), und somit der Oberseite (2a) und Unterseite (2b) sowie von optionalen Seitenelementen, beträgt beispielsweise 0.1 - 3 mm, insbesondere 0.1 - 1 mm, gemessen gemäss EN ISO 16809:2019.

In einer bevorzugten Ausführungsform des Wärmekissens (1) ist an der Aussenhülle (2), insbesondere an der Oberseite (2a) der Aussenhülle (2), ein Flüssigkristall-Thermometer (7) angebracht ist, wobei das Flüssigkristall-Thermometer (7)
- einen Temperatur-Anzeigebereich von mindestens 40°C bis 100°C, insbesondere von mindestens 60°C bis 80°C, aufweist, und/oder
- ein thermochromatisches Flüssigkristall-Thermometer (7) ist, wobei der Farbumschlag bevorzugt zwischen 40°C bis 100°C, insbesondere von mindestens 60°C bis 80°C, erfolgt.
Dies ermöglicht eine schnelle visuelle Temperaturbestimmung des Wärmekissens (1) und somit des Wärmemediums (3). Dies ist von grossem Vorteil, da die Temperaturbestimmung nicht von Hand gemacht werden muss. Denn eine zulange Berührung eines heissen Wärmekissens kann zu Verbrennungen führen kann. Daher ist die Anwesenheit eines Flüssigkristall-Thermometers (7) beispielsweise für Personen mit eingeschränkter Sensibilität an den Händen äusserst vorteilhaft.

In einer weiteren bevorzugen Ausführungsform des Wärmekissens (1) ist das Wärmepad (6, 6*) im Wärmekissen (1) im Wesentlichen vollständig vom Wärmemedium (3) umgeben, und somit nicht fest mit der Aussenhülle (2) verbunden. Somit kann sich das Wärmepad (6, 6*) im Wärmekissen (1) im Wesentlichen frei bewegen, insbesondere wenn das Wärmemedium (3) in flüssiger Form vorliegt. Somit kann sich das Wärmepad (6, 6*) bei dessen Erwärmung innerhalb des Wärmekissens (1) nach oben bewegen, wodurch bei Überschreiten einer gewissen Distanz das Wärmepad (6, 6*) nicht mehr erwärmt wird. Dies verhindert ein zu schnelles Erhitzen des Wärmekissens (1) und ermöglicht eine bessere Überwachung des Wärmevorgangs.

In einer ganz besonders bevorzugten Ausführungsform ist das erfindungsgemässe Wärmekissen (1) umfassend das Wärmekissen (6) und/oder das erfindungsgemässe Wärmepad (6*) mit einer Kraft von maximal 1 N zerstörungsfrei und reversibel um ein Rohr mit einem Durchmesser von 10 cm, insbesondere mit einem Durchmesser von 5 cm, biegbar. Ist das Wärmemedien (3) bei Raumtemperatur (23°C) flüssig oder rieselfähig, gilt für die genannte Biegbarkeit Raumtemperatur, d.h. 23°C. Ist das Wärmemedien (3) bei Raumtemperatur fest und schmilzt beispielsweise zwischen 20°C und 80°C, wird die Biegbarkeit bevorzugt 10°C oberhalb der Schmelztemperatur bestimmt. Aufgrund dieser Biegbarkeit passen sich das Wärmekissen (1) und das Wärmepad (6,6*) im Wesentlichen jeder beliebigen Form an. Dadurch verletzt das Wärmepad (6,6*) keine benachbarten Materialien wie die Aussenhaut (2) des Wärmekissens (1).

Die genannte zerstörungsfreie und reversible Biegbarkeit wird durch eine geeignete Auswahl der Aussenhülle (2), des ferromagnetischen Elements (4, 4a, 4b) und gegebenenfalls des polymerhaltigen Materials (5) erzielt. Der Fachmann kann die geeignete Auswahl der Materialien ohne erfinderische Tätigkeit erzielen. Zudem ist es hilfreich, wenn die Aussenhülle (2) nicht mehr als etwa 99 Vol.-% mit Wärmemedium (3) gefüllt ist. Durch die genannte zerstörungsfreie und reversible Biegbarkeit kann das Wärmepad (6, 6*) beispielsweise einfach zusammengerollt werden und in bestehende Wärmekissen ohne Wärmepad in die Öffnung des Wärmepads eingefügt werden. Auch kann das Wärmepad (6, 6*) einfach transportiert werden, ohne dass es zerbricht oder andere Artikel zerstört. Besonders vorteilhaft ist es, dass das Wärmepad (6, 6*) wie auch das Wärmekissen (1) sich aufgrund der hohen Biegbarkeit der Umgebung - beispielsweise einer Körperstelle, auf die sie gelegt werden - äusserst gut anpassen. Dadurch entstehen auch bei empfindlicher Haut keine Rötung und kein Dekubitus.

### Das Wärmepad (6, 6*)

Das Wärmepad (6) ist im erfindungsgemässen Wärmekissen (1) angeordnet und wird durch das ferromagnetische Element (4), gegebenenfalls zusammen mit dem Material (5), gebildet, wobei das ferromagnetische Element (4)
- zumindest teilweise mit einem polymerhaltigen Material (5) umgeben ist;
- mindestens zwei, bevorzugt 2 bis 20, insbesondere 3 bis 10, übereinander angeordnete ferromagnetische Folien (4a) umfasst, die miteinander verbunden sind; und/oder
- in Form eines Textils vorliegt enthaltend sich berührende ferromagnetische Metallfasern (4b).

Das erfindungsgemässe Wärmepad (6*) ist speziell geeignet für den Einsatz im erfindungsgemässen Wärmekissen (1) und kann daher unabhängig vom Wärmekissen (1) vorliegen. So kann es als solches transportiert, erwärmt und verwendet werden. Zudem kann mit dem Wärmepad (6*) auch ad-hoc ein Wärmekissen (1) erstellt werden, indem es beispielsweise zusammen mit einem Wärmemedium wie Wasser in einen Plastiksack gefüllt und dieser verschlossen wird.

Das Wärmepad (6*) umfasst
- ein ferromagnetisches Element (4) in Form von mindestens zwei, bevorzugt von 2 bis 20, insbesondere von 3 bis 10, übereinander angeordneten ferromagnetischen Folien (4a), wobei der Randbereich der Folien (4a) mit mindestens je einer Schicht des Materials (5) zumindest teilweise umfasst ist und das Material (5) und die Folien (4a) punktuell oder vollständig miteinander verbunden sind, oder
- ein Textil mit sich berührenden ferromagnetischen Metallfasern (4b).

Das Wärmepad (6, 6*) umfasst in einer bevorzugten Ausführungsform übereinander angeordneten ferromagnetischen Folien (4a), wobei
- der Randbereich der Folien (4a) von beiden Aussenseiten der übereinander angeordneten Folien (4a) mit mindestens je einer Schicht des Materials (5) zumindest teilweise umfasst und das Material (5) und die Folien (4a) miteinander punktuell verbunden sind, wodurch das Wärmemedium (3) zwischen den Schichten des Materials (5) hindurch zwischen zumindest zwei Folien (4a) gelangen kann,
- das Material (5) den Randbereich der Folien (4a), gegebenenfalls vollständig, umfasst und das Material (5) und die Folien (4a), gegebenenfalls vollständig, miteinander verbunden, sind, wobei mindestens eine Oberfläche der Folien (4a) Öffnungen (7) aufweist, durch welche das Wärmemedium (3) zwischen die zumindest zwei Folien (4a) gelangen kann, oder
- das Material (5) den Randbereich der Folien (4a) vollständig umgibt und das Material (5) und die Folien (4a) vollständig miteinander verbunden, sind, wobei zwischen mindestens zwei Folien (4a) ein Wärmemedium (3) angeordnet ist. Dabei ist es in dieser Ausführungsform besonders vorteilhaft, wenn das Material (5) und/oder die Folien (4a) aufgrund von deren Anordnung resp. deren Dehnbarkeit ausdehnen kann. Dies ist besonders beim Verdampfen von Wärmemedium (3) wichtig, damit sich das Volumen zwischen den Folien (4a) vergrössern kann, was zu geringerer Dichte und verstärktem Auftrieb führt.

Die Verbindung des Materials (5) mit dem Randbereich der Folien (4a) - ob punktuell oder vollständig - erfolgt vorteilhafterweise mittels Verklebung, Verschweissung und/oder in dem sie zusammen vernäht werden. Dabei kann die Verklebung direkt mit dem Material (5) erfolgen, was insbesondere vorteilhaft ist, wenn das Material (5) bei der Anwendung in flüssigem oder pastösem Zustand ist. Nicht-limitierende Beispiele dazu geeigneter Materialien (5) sind und Polymere Materialien (5) wie Duromere, Duroplaste, Kunstharze, Polyesterharze, Epoxidharze, Thermoplaste, und Elastomere wie Silikone und Siloxane. Alternativ erfolgt die Verklebung mit einem handelsüblichen Klebstoff, was insbesondere voreilhaft ist, wenn das Material (5) ein bei der Anwendung des Materials (5) in fester Form vorliegt. Solche Materialien (5) sind beispielsweise Kork, Leder, vulkanisierter und somit ausgehärteter Kautschuk, sowie vorgefertigte und ausgehärtete Polymere. Alternativ - oder zusätzlich - werden diese Materialien miteinander vernäht.

In einer Ausführungsform werden die übereinander angeordneten ferromagnetischen Folien (4a) miteinander verschweisst und das Material (5) mit den verschweissten Folien verklebt und/oder vernäht.

Weisen die Oberflächen der Folien (4a) Öffnungen (7) auf, können diese beispielsweise einen Durchmesser von etwa 0.5-5 mm aufweisen und/oder rund, eckig, rechteckig oder länglich in Form von Schlitzen sein.

Umfasst das Wärmepad (6*) ein Textil mit sich berührenden ferromagnetischen Metallfasern (4b) und weiteren Fasern, basieren die weiteren Fasern des Textils bevorzugt auf einer oder mehreren Naturfasern, einer oder mehreren Chemiefasern, insbesondere Fasern aus natürlichen Polymeren wie Cellulosefasern, Wollfasern und/oder Baumwollfasern, einer oder mehreren synthetischen Fasern, beispielsweise basierend auf Polyester, Polyamid, Aramid, Nylon, Polyurethan und/oder Elastan.

### Das Verfahren

Beim erfindungsgemässen Verfahren zum induktiven Erwärmen eines Wärmemediums (3) mit dem erfindungsgemässen Wärmepad (6*) wird
- das Wärmepad (6*) in eine Aussenhülle (2) gebracht, wobei vorher, nachher und/oder gleichzeitig Wärmemedium (3) in die Aussenhüllte (2) gefüllt wird, und
- die Aussenhülle (2) mit Wärmepad (6*) und Wärmemedium (3) auf einer Induktionsheizplatte, insbesondere mittels Anlegen eines Wechselstroms an der Induktionsheizplatte, erwärmt wird, wobei
- die Aussenhülle (2), gegebenenfalls verschlossen wird, wodurch so das erfindungsgemässe Wärmekissen (1) erhalten wird. Dabei erfolgt das Verschliessen der Aussenhülle (2) bevorzugt vor dem Erwärmen des Wärmekissens (1), wobei das Verschliessen reversibel oder irreversibel erfolgen kann.

Wird die Aussenhülle (2) nicht verschlossen, wird zwar kein Wärmekissen (1) erhalten, es kann jedoch mit dem erfindungsgemässen Verfahren das Wärmemedium (3) erwärmt werden. In dieser Ausführungsform ist das Wärmemedium (3) bevorzugt Wasser, welches so auf einfache Art und Weise mit dem Wärmepad (6*) auf einem Induktionsherd erwärmt werden kann. Dabei kann die Aussenhülle (2) beispielsweise ein Plastiksack oder ein Gefäss aus Kunststoff, Keramik oder Glas sein.

In einer bevorzugten Ausführungsform des Verfahrens wird das Wärmemedium (3) - und somit das Wärmekissen (1) mittels Anlegen eines Wechselstroms
- mit einer Frequenz von 25 bis 50 kHz,
- während 1 bis 60 Minuten, insbesondere während 3 bis 15 Minuten, und/oder
- bei einer Leistung von1 kW bis 10 kW, insbesondere von 3 kW bis 6 kW
erwärmt, beispielsweise auf eine Temperatur von 80°C (?).

So kann beispielhaft ein Wärmekissen (1) mit einer Aussenhülle (2) Oberseite (2a) und Unterseite (2b) aus Weich-PVC mit einer Schichtdicke von 0.3 mm, einer Länge von 23 cm und einer Breite von 14 cm, mit 7.5 dl Wasser als Wärmemedium (3) und einem Wärmepad (6) umfassend 6 zusammen vernähte ferromagnetische Metallfolien mit einer Schichtdicke von je 0.1mm auf einem Induktionsherd auf Heizstufe 6 von insgesamt 10 Stufen mit einer maximalen Leistung von 7.5 kWatt und einer Frequenz im Bereich von 25-50 kHz in 4 Minuten auf eine Temperatur von 85 °C erwärmt werden.

### Die Verwendung

Das erfindungsgemässe Wärmekissens (1) wird erfindungsgemäss verwendet zum Auftauen, Wärmen und/oder Warmhalten von Substraten wie Lebensmittel, zum Wärmen und/oder Warmhalten von Körperstellen von lebenden Körpern, und/oder zur Wärmebehandlung in Spitälern, medizinischen Praxen und/oder im privaten Bereich, wobei das Wärmekissen (1) gegebenenfalls auch mit einer Schutzhülle umgeben sein kann. Ist beispielsweise der flächige Boden eines Stoffbehälters mit einem Wärmekissen (1) ausgerüstet, kann dieses bequem auf einem Induktionsherd erwärmt werden. Anschliessend können gefrorene Backwaren wie Brötchen in den Stoffbehälter mit erwärmtem Wärmekissen (1) gelegt werden, wobei dies langsam auftauen. Dabei wurde überraschenderweise gefunden, dass die aufgetauten Brötchen auch noch nach einer Stunde warm und nicht ausgetrocknet sind.

Dabei basiert die Schutzhülle bevorzugt auf einem Textil, Gewebe, Vlies, Leder und/oder Kunstleder. Eine besonders bevorzugte Ausführungsform der Schutzhülle ist in der EP-A-3 718 519 beschrieben.

Das erfindungsgemässe Wärmepad (6*) wird vorteilhafterweise zum Erwärmen eines Wärmemediums (3) verwendet, wobei das Wärmemedium (3) bevorzugt Wasser ist. Dabei kann das Wärmemedium (3) Teil des Wärmekissens (1) sein oder in einer offenen Aussenhülle (2) erwärmt werden. Ist das Wärmemedium (3) Wasser, kann das erhaltene Warm- oder Heisswasser beispielsweise beim Campieren zum Duschen, Wäschewaschen und/oder Kochen verwendet werden.

Das Wärmepad (6*) kann zudem zum Nachrüsten von bestehenden Wärmekissen mit einer Öffnung verwendet werden. So kann das Wärmepad (6*) auf einfache Art und Weise zusammengerollt werden und durch die Öffnung des Wärmekissens geführt werden, wodurch - wenn mit Wärmemedium (3) gefüllt, das erfindungsgemässe Wärmkissen (1) erhalten wird.

Alternativ kann das Wärmepad (6*) selbst erwärmt und als warmes Wärmepad (6*) anstelle eines Wärmekissens (1) verwendet werden. Für diese Ausführungsform ist besonders vorteilhaft, wenn das Wärmepad (6*) ein Textil umfasst mit sich berührenden ferromagnetischen Metallfasern (4b) und weiteren Fasern, insbesondere auf einer oder mehreren Naturfasern, einer oder mehreren Chemiefasern, insbesondere Fasern aus natürlichen Polymeren wie Cellulosefasern, Wollfasern und/oder Baumwollfasern, einer oder mehreren synthetischen Fasern, beispielsweise basierend auf Polyester, Polyamid, Aramid, Nylon, Polyurethan und/oder Elastan.

Das Wärmepad (6*) kann beispielsweise auch in Kleidungsstücke, zum Beispiel Handschuhe, eingearbeitet werden, wodurch das Kleidungsstück zum Wärmekissen (1) werden kann. Dabei kann beispielsweise die Aussenschicht des Handschuhs die Aussenhülle (2) bilden und als Wärmemedium (3) kann ein Textil verwendet werden, in welches das Wärmepad (6*) - beispielsweise in Form eines Textils - umgibt.

Es werden folgende Bezugszeichen verwendet:
- 1: Wärmekissen
- 2: Aussenhülle
- 2a: Oberseite der Aussenhülle
- 2b: Unterseite der Aussenhülle
- 3: Wärmemedium
- 4: ferromagnetisches Element
- 4a: ferromagnetische Folien
- 4b: sich berührende ferromagnetische Metallfasern
- 5: polymerhaltiges Material
- 6: Wärmepad
- 6*: erfindungsgemässes Wärmepad
- 7: Öffnungen

Im Folgenden werden nicht-limitierende, bevorzugte Ausführungsformen des erfindungsgemässen Wärmekissens (1) und des erfindungsgemässen Wärmepads (6*) anhand der nachfolgenden Zeichnungen beschrieben. Diese sind nicht einschränkend auszulegen und werden als Bestandteil der Beschreibung verstanden:

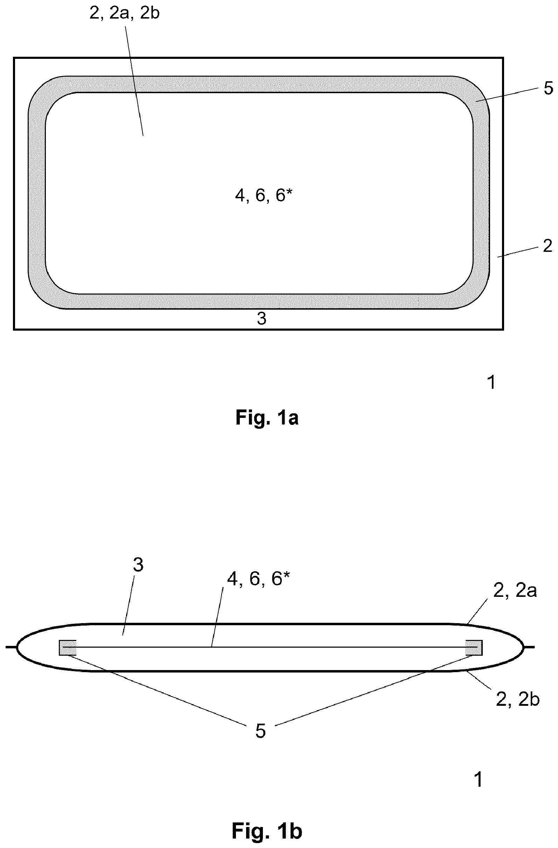

- Fig. 1a: zeigt beispielhaft die Aufsicht eines Wärmekissens (1) mit rechteckiger Form mit Aussenhülle (2), Oberseite (2a) und Unterseite (2b) sowie einem Wärmemedium (3). Darin eingebettet ist das Wärmepad (6, 6*) umfassend das ferromagnetische Element (4), welches mit einem polymerhaltigen Material (5) umgeben ist.
- Fig. 1b: zeigt den Querschnitt des Wärmekissens (1) von Fig. 1a mit Aussenhülle (2), Oberseite (2a) und Unterseite (2b), wobei die Oberseite (2a) und Unterseite (2b) im Randbereich zusammengeschweisst sind. In der Aussenhülle (2) ist das Wärmemedium (3) angeordnet, in welchem das Wärmepad (6, 6*) umfassend das ferromagnetische Element (4), welches mit einem polymerhaltigen Material (5) umgeben ist, eingebettet ist. Somit ist das Wärmepad (6, 6*) innerhalb des Wärmekissens (1) frei bewegbar. Das Wärmepad (6, 6*) kann ein Wärmepad (6) sein und somit kann das ferromagnetische Element (4) beispielsweise einschichtig sein. Das ferromagnetische Element (4) kann auch mindestens zwei übereinander angeordnete ferromagnetische Folien (4a) umfassen, die - beispielsweise im Randbereich und zusammen mit dem polymeren Material (5), welches das Element (4) vollständig umrandet - miteinander verbunden sind. In einer anderen Ausführungsform liegt das ferromagnetische Element (4) in Form eines Textils vor, enthaltend sich berührende ferromagnetische Metallfasern (4b).

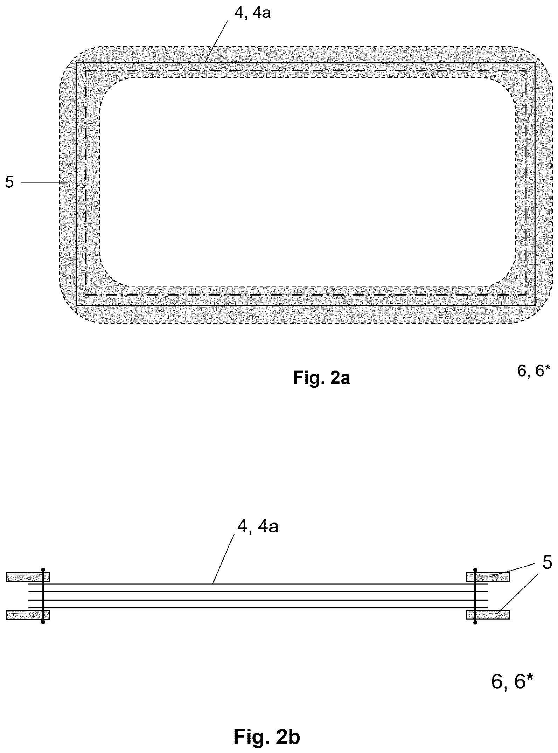

- Fig. 2a: zeigt beispielhaft die Aufsicht eines Wärmepads (6,6*) umfassend als ferromagnetisches Element (4) übereinander angeordnete ferromagnetische Folien (4a), welche im Randbereich zusammen mit dem polymeren Material (5) vernäht sind.
- Fig. 2b: zeigt den Querschnitt des Wärmepads (6,6*) von Fig. 2a mit insgesamt vier übereinander angeordneten Folien (4a). Im Randbereich der Folien (4a) ist ober- und unterhalb der Folien polymeres Material (5), welches mit den Folien (4a) zusammen vernäht sind. Somit kann Wärmemedium (3) von der Seite zwischen die Folien (4a) gelangen. Beim Erwärmen des Wärmekissens (1) mit dem Wärmepad (6,6*) wird somit das zwischen den Folien (4a) befindliche Wärmemedium (3) besonders schnell erhitzt.

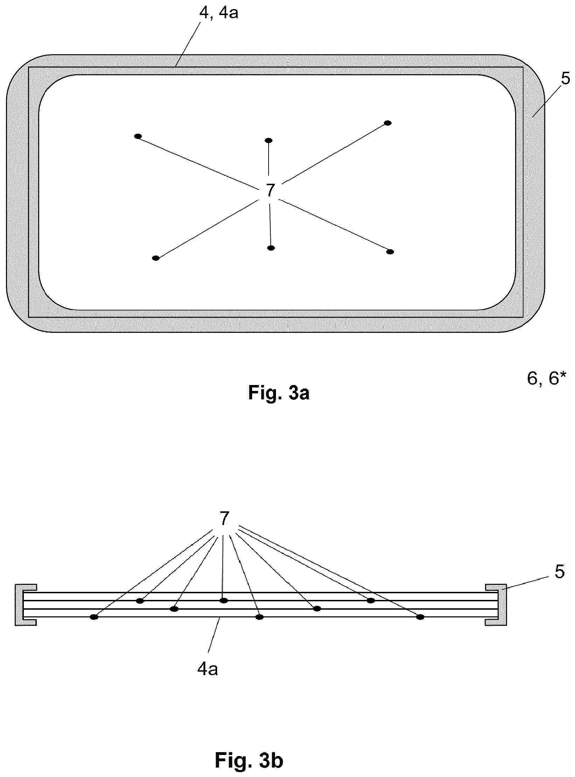

- Fig. 3a: zeigt beispielhaft die Aufsicht eines Wärmepads (6,6*) umfassend als ferromagnetisches Element (4) übereinander angeordnete ferromagnetische Folien (4a), welche im Randbereich mit polymerem Material (5) vollständig umgeben sind. Dabei weisen Folien (4a) kleine Öffnungen (7) auf, durch welche Wärmemedium (3) zwischen die Folien (4a) gelangen kann, wodurch es schnell erwärmt werden kann.
- Fig. 3b: zeigt den Querschnitt des Wärmepads (6,6*) von Fig. 3a mit insgesamt vier übereinander angeordneten Folien (4a), welche im Randbereich der Folien (4a) vollständig von polymerem Material (5) umgeben sind. Bis auf die oberste Folie (4a) weisen alle Folien (4a) kleine Öffnungen auf, durch welche Wärmemedium (3) zwischen die Folien (4a) gelangen kann, was eine schnelle Erwärmung des Wärmemediums (3) erlaubt.

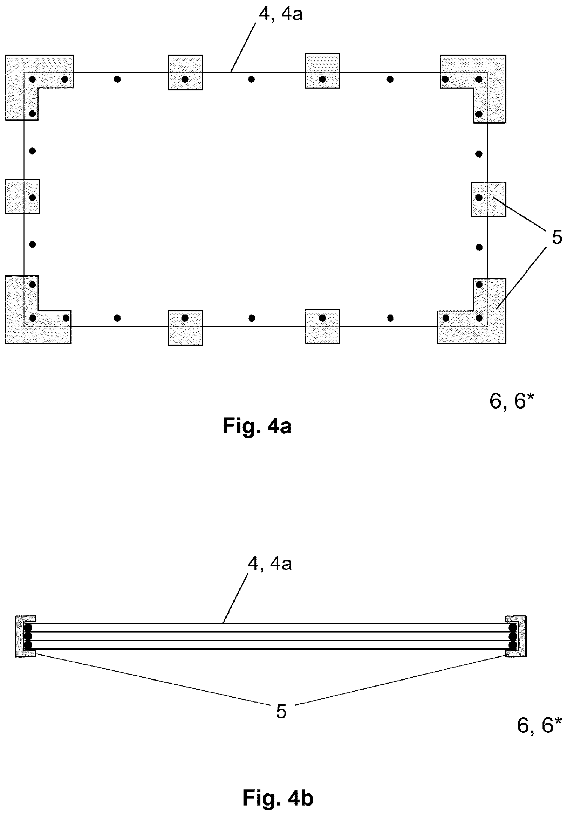

- Fig. 4a: zeigt beispielhaft die Aufsicht eines Wärmepads (6,6*) umfassend als ferromagnetisches Element (4) übereinander angeordnete ferromagnetische Folien (4a), welche im Randbereich mit polymerem Material (5) teilweise umgeben sind. Die Folien (4a) sind im Randbereich miteinander punktuell mittels Verschweissen und/oder Verkleben verbunden. Dabei umfasst das polymere Material (5) - wo angeordnet - alle Folien (4a). Dabei kann das Wärmemedium (3) seitlich zwischen dem polymeren Material (5) und zwischen den Bereichen, wo die Folien (4a) miteinander verschweisst und/oder verklebt sind, in die Zwischenräume der Folien fliessen.
- Fig. 4b: zeigt den Querschnitt des Wärmepads (6,6*) von Fig. 4a mit insgesamt vier übereinander angeordneten Folien (4a), welche im Randbereich der Folien (4a) punktuell miteinander verschweisst und/oder verklebt und von polymerem Material (5) umgeben sind.

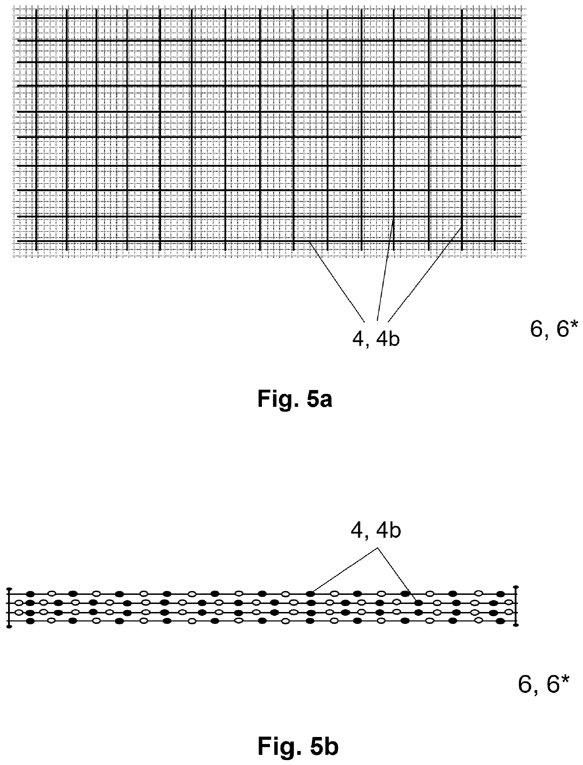

- Fig. 5a: zeigt beispielhaft die Aufsicht eines Wärmepads (6,6*) umfassend das ferromagnetische Element (4) in Form eines Textils enthaltend sich berührende ferromagnetische Metallfasern (4b). Zwischen den Metallfasern (4b) sind weitere, nicht-metallische Fasern in das Textil hinein gewoben.
- Fig. 5b: zeigt den Querschnitt des Wärmepads (6,6*) von Fig. 5a mit insgesamt vier übereinander angeordneten Textilschichten umfassend sich berührende ferromagnetische Metallfasern (4b, schwarz dargestellt). Zwischen den Metallfasern (4b) sind nichtmetallische Fasern (weiss gefüllte ovale Bereiche) eingewoben. Die übereinander angeordneten Textilschichten sind im Randbereich miteinander vernäht.

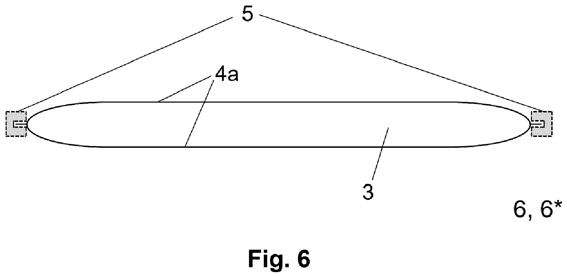

- Fig. 6: zeigt beispielhaft den Querschnitt eines Wärmepads (6,6*) mit zwei übereinander angeordneten ferromagnetischen Folien (4a), die ein Wärmemedium (3), zusammen mit Luft (nicht dargestellt), umschliessen. Im Randbereich weisen die Folien (4a) ein polymeres Material (5) auf. Dabei können sich das Material (5) und/oder die Folien (4a) aufgrund der Beschaffenheit und/oder Dehnbarkeit ausdehnen kann, damit sich das Volumen zwischen den Folien (4a) beim Verdampfen von Wärmemedium (3) vergrössert, um den Auftrieb des Wärmepads (6,6*) zu erhöhen.

## Patentansprüche

1. Mittels Induktion heizbares Wärmekissen (1) umfassend eine Aussenhülle (2) mit Oberseite (2a) und Unterseite (2b), ein Wärmemedium (3) und ein ferromagnetisches Element (4), wobei das Wärmemedium (3) durch die Aussenhülle (2) begrenzt ist und das ferromagnetische Element (4) im Wärmemedium (3) angeordnet ist, **dadurch gekennzeichnet, dass** das ferromagnetische Element (4)
- zumindest teilweise mit einem polymerhaltigen Material (5) umgeben ist,
- mindestens zwei übereinander angeordnete ferromagnetische Folien (4a) umfasst, die miteinander verbunden sind, und/oder
- in Form eines Textils vorliegt enthaltend sich berührende ferromagnetische Metallfasern (4b),
wodurch das ferromagnetische Element (4), gegebenenfalls zusammen mit dem Material (5), ein Wärmepad (6) bildet.

2. Wärmekissen (1) nach Anspruch 1, **dadurch gekennzeichnet, dass** das ferromagnetische Element (4), die ferromagnetische Folie (4a) und/oder die ferromagnetischen Metallfasern (4b) auf Eisen, Nickel, Cobalt, Chromdioxid und/oder einer ferromagnetischen Legierung, insbesondere auf AlNiCo, SmCo, Nd₂Fe₁₄B, Ni₈₀Fe₂₀ oder NiFeCo basiert, wobei gegebenenfalls die ferromagnetische Folie (4a) als metallische Folie vorliegt, die faserverstärkt ist, und/oder in Form einer Kunststofffolie, welche mit einer ferromagnetischen Folie, beispielsweise einer selbstklebenden Eisenfolie, beschichtet ist.

3. Wärmekissen (1) nach Anspruch 1 oder 2, **dadurch gekennzeichnet, dass** die übereinander angeordneten ferromagnetischen Folien (4a) übereinander geschichtet und miteinander verbunden, bevorzugt punktuell und/oder im Randbereich miteinander verbunden, insbesondere miteinander verschweisst, verklebt und/oder vernäht, sind.

4. Wärmekissen (1) nach mindestens einem der Ansprüche 1 bis 3, **dadurch gekennzeichnet, dass**
- die ferromagnetischen Folien (4a) eine Dicke von je 0.01 bis 5 mm, bevorzugt von 0.05 bis 3 mm, insbesondere von 0.1 bis 1 mm, gemessen gemäss EN ISO 16809:2019, und/oder
- die ferromagnetischen Metallfasern (4b) einen Durchmesser von 0.2 µm bis 3 mm, insbesondere 0.5 µm bis 2 mm, aufweisen.

5. Wärmekissen (1) nach mindestens einem der Ansprüche 1 bis 4, **dadurch gekennzeichnet, dass** das optionale polymerhaltige Material (5) auf Basis eines Polymers, Silikons; Siloxans, von Kork, Leder und/oder Kautschuk ist, wobei das Polymer auch geschäumt und/oder faserverstärkt sein kann und bevorzugt ein Duromer, Duroplast, Kunstharz, Polyesterharz, Epoxidharz, Elastomer und Thermoplast umfasst.

6. Wärmekissen (1) nach mindestens einem der Ansprüche 1 bis 5, **dadurch gekennzeichnet, dass** das Wärmemedium (3)
- Wasser, oder eine wässrige Lösung;
- eine organische Verbindung mit Schmelzpunkt unterhalb 20°C, insbesondere ein Glykol, Polyethylenglykol, Polypropylenglykol und/oder Paraffin;
- eine organische Verbindung mit Schmelzpunkt zwischen 20°C und 80°C, bevorzugt zwischen 20°C und 60°C, insbesondere zwischen 30°C und 50°C, wie z.B. Paraffine, Alkohol, Glykol, Polyol, Zucker, Keton, Ester, Ether, Carbonsäure, Fettsäure, Amid, eine Schwefel-, Phosphor- und/oder Stickstoff-Verbindung; und/oder
- Sand, Fruchtkerne wie Kerne von Steinobst und Kernobst und/oder ein Textil
umfasst.

7. Wärmekissen (1) nach mindestens einem der Ansprüche 1 bis 6, **dadurch gekennzeichnet, dass** die Aussenhülle (2) mit Oberseite (2a) und Unterseite (2b) auf einem Polymer oder einem polymerbeschichteten Textil, Gewebe oder Vlies basiert, wobei das Polymer und/oder die Polymerbeschichtung bevorzugt auf Basis von Polyethylen (PE), Polypropylen (PP), Polyethylenterephthalat (PET), Polyvinylchlorid (PVC), Polyvinylidenchlorid (PVDC), Ethylen-Propylen-Dien-Kautschuk (EPDM), Chloropren-Kautschuk (CR), Styrol-Butadien-Kautschuk (SBR), Polyurethan (PU), Polyamid (PA), deren Misch-, Co- und Block(co)polymere und/oder deren Derivate ist.

8. Wärmekissen (1) nach mindestens einem der Ansprüche 1 bis 7, **dadurch gekennzeichnet, dass** an der Aussenhülle (2), insbesondere an der Oberseite (2a) der Aussenhülle (2), ein Flüssigkristall-Thermometer (7) angebracht ist, wobei das Flüssigkristall-Thermometer (7)
- einen Temperatur-Anzeigebereich von mindestens 40°C bis 100°C, insbesondere von mindestens 60°C bis 80°C, aufweist, und/oder
- ein thermochromatisches Flüssigkristall-Thermometer (7) ist, wobei der Farbumschlag bevorzugt zwischen 40°C bis 100°C, insbesondere von mindestens 60°C bis 80°C, erfolgt.

9. Wärmekissen (1) nach mindestens einem der Ansprüche 1 bis 8, **dadurch gekennzeichnet, dass** das Wärmepad (6) im Wärmekissen (1) im Wesentlichen vollständig vom Wärmemedium (3) umgeben ist, und somit nicht fest mit der Aussenhülle (2) verbunden ist.

10. Wärmepad (6*) für Wärmekissen (1) nach mindestens einem der Ansprüche 1 bis 9, **dadurch gekennzeichnet, dass** das Wärmepad (6*)
- ein ferromagnetisches Element (4) in Form von mindestens zwei, übereinander angeordneten ferromagnetischen Folien (4a) umfasst, wobei der Randbereich der Folien (4a) mit mindestens je einer Schicht des Materials (5) zumindest teilweise umfasst ist und das Material (5) und die Folien (4a) punktuell oder vollständig miteinander verbunden sind, oder
- ein Textil umfasst mit sich berührenden ferromagnetischen Metallfasern (4b).

11. Wärmepad (6*) nach Anspruch 10, **dadurch gekennzeichnet, dass**
- der Randbereich der Folien (4a) von beiden Aussenseiten der übereinander angeordneten Folien (4a) mit mindestens je einer Schicht des Materials (5) zumindest teilweise umfasst ist und das Material (5) und die Folien (4a) miteinander punktuell verbunden, sind, wodurch das Wärmemedium (3) zwischen den Schichten des Materials (5) hindurch zwischen zumindest zwei Folien (4a) gelangen kann,
- das Material (5) den Randbereich der Folien (4a), gegebenenfalls vollständig, umfasst und das Material (5) und die Folien (4a), gegebenenfalls vollständig, miteinander verbunden, sind, wobei mindestens eine Oberfläche der Folien (4a) Öffnungen (7) aufweist, durch welche das Wärmemedium (3) zwischen die zumindest zwei Folien (4a) gelangen kann, oder
- das Material (5) den Randbereich der Folien (4a) vollständig umgibt und das Material (5) und die Folien (4a) vollständig miteinander verbunden, sind, wobei zwischen mindestens zwei Folien (4a) Wärmemedium (3) angeordnet ist.

12. Wärmekissen (1) nach mindestens einem der Ansprüche 1 bis 9 und Wärmepad (6*) nach Anspruch 10 oder 11, **dadurch gekennzeichnet, dass** das Wärmekissen (1) und/oder das Wärmepad (6*) mit einer Kraft von maximal 1 N zerstörungsfrei und reversibel um ein Rohr mit einem Durchmesser von 10 cm, insbesondere mit einem Durchmesser von 5 cm, biegbar sind.

13. Verfahren zum induktiven Erwärmen eines Wärmemediums (3) mit dem Wärmepad (6*) nach mindestens einem der Ansprüche 10 bis 12, **dadurch gekennzeichnet, dass**
- das Wärmepad (6*) in eine Aussenhülle (2) gebracht wird, wobei vorher, nachher und/oder gleichzeitig Wärmemedium (3) in die Aussenhüllte (2) gefüllt wird, und
- die Aussenhülle (2) mit Wärmepad (6*) und Wärmemedium (3) auf einer Induktionsheizplatte, insbesondere mittels Anlegen eines Wechselstroms an der Induktionsheizplatte, erwärmt wird, wobei
- die Aussenhülle (2), gegebenenfalls verschlossen wird, wodurch so das Wärmekissen (1) nach mindestens einem der Ansprüche 1 bis 9 erhalten wird.

14. Verfahren nach Anspruch 13, **dadurch gekennzeichnet, dass** das Wärmemedium (3), und somit das Wärmekissen (1), mittels Anlegen eines Wechselstroms
- Mit einer Frequenz von 25 bis 50 kHz,
- während 1 bis 60 Minuten, insbesondere während 3 bis 15 Minuten, und/oder
- bei einer Leistung von 1 kW bis 10 kW, insbesondere von 3 bis 6 kW
erwärmt wird, beispielsweise auf eine Temperatur von 80°C (?).

15. Verwendung des Wärmekissens (1) nach mindestens einem der Ansprüche 1 bis 9 und 12 zum Auftauen, Wärmen und/oder Warmhalten von Substraten wie Lebensmittel, zum Wärmen und/oder Warmhalten von Körperstellen von lebenden Körpern, und/oder zur Wärmebehandlung in Spitälern, medizinischen Praxen und/oder im privaten Bereich, wobei das Wärmekissen (1) gegebenenfalls auch mit einer Schutzhülle umgeben sein kann.
